# EUROPEAN PATENT SPECIFICATION

(11) **EP 1 687 310 B1**
(45) Date of publication and mention of the grant of the patent: **12.12.2007**
(21) Application number: 04796303.8
(22) Date of filing: 25.10.2004
(51) Int. Cl.: C07D 489/02

(54) **METHOD FOR THE CATALYTIC PRODUCTION OF HYDROCODONE AND HYDROMORPHONE**
VERFAHREN ZUR KATALYTISCHEN HERSTELLUNG VON HYDROCODON UND HYDROMORPHON
PROCÉDÉ POUR LA PRODUCTION CATALYTIQUE D'HYDROCODONE ET D'HYDROMORPHONE

(30) Priority: 05.11.2003 WO PCT/US03/35462; 13.05.2004 US 495503
(43) Date of publication of application: 09.08.2006
(73) Proprietor: MALLINCKRODT INC., St. Louis, MO 63134 (US)
(72) Inventor: WANG, Peter Xianqi, Chesterfield, Missouri 63017 (US); WHITE, Carl Ray, St. Louis, Missouri 63122 (US)
(74) Representative: Cockerton, Bruce Roger
(86) International application number: PCT/US2004/035292
(87) International publication number: WO 2005/113557

(56) References cited:
- WO-A-98/05667
- US-A- 2 654 756
- NINAN, ALEYAMMA ET AL: "An improved synthesis of noroxymorphone" TETRAHEDRON , 48(32), 6709-16 CODEN: TETRAB; ISSN: 0040-4020, 1992, XP002285612

## Description

### BACKGROUND OF THE INVENTION

The present invention relates to a method for the catalytic production of hydrocodone, hydromorphone, and analogs thereof, and more particularly to a method for catalytically converting codeine and morphine into hydrocodone and hydromorphone, respectively.

Hydrocodone and hydromorphone are opioid analgesics having similar qualities to codeine and morphine. Conventional methods for producing hydrocodone and hydromorphone typically involve a two step oxidation/reduction route from codeine and morphine. Unfortunately, these methods are expensive and inefficient. Attempts to improve efficiency have included the use of catalytic methods. Known methods include the use of metallics, typically Ru, Rh, Pd and Pt on activated carbon as well as metallic complexes. The preparation of these catalysts is difficult, yields are poor, and isolation of the product is often burdensome.

Other catalytic methods, including the use of finely-divided platinum or palladium in an acidic media, are environmentally undesirable. Enzymatic methods of conversion have also been attempted, but as with the methods of catalysis discussed above, these methods are costly and difficult to scale up.

There is therefore a need for an improved method of conversion that is easily scaled up and economical for manufacturing purposes.

### SUMMARY OF THE INVENTION

An aspect of the present invention is to provide a method for the catalytic conversion of a compound of Formula I into a compound of Formula II utilizing at least one transition metal complex as catalyst, 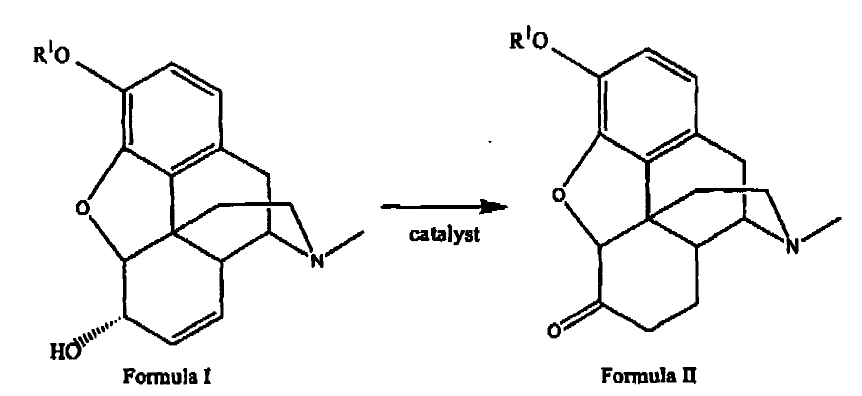 wherein R¹ is H, alkyl, aryl or acyl,

In one aspect of the present invention the transition metal complex is of the formula [M(PR³R⁴R⁵)ₙXₘ]ₚ; wherein M is a Group 8, 9 or 10 transition metal; R³, R⁴ and R⁵ are selected from the group consisting of alkyl, aryl, alkoxyl, phenoxyl and combinations thereof; X is an anion selected from the group consisting of CHO₂, CH₃CO₂, CF₃CO₂, ArCO₂, CH₃SO₃, p-tolylSO₃, HSO₄ and H₂PO₄; n is 1, 2, 3 or 4; m is 1 or 2; and p is at least 1.

Another aspect of the present invention is to provide a one step method for the conversion of codeine or morphine into hydrocodone or hydromorphone, respectively.

These are merely illustrative aspects of the present invention and should not be deemed an all-inclusive listing of the innumerable aspects associated with the present invention. These and other aspects will become apparent to those skilled in the art in light of the following disclosure.

### DETAILED DESCRIPTION

There is provided a method for the conversion of a compound according to Formula I to a compound according to Formula II, 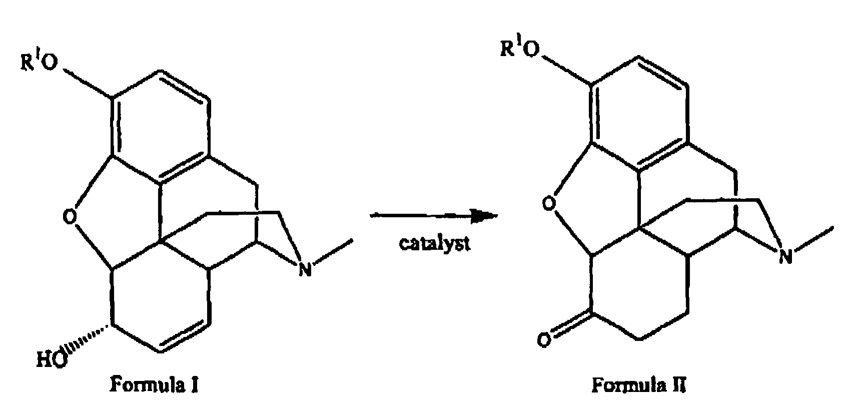 wherein R¹ is H, alkyl, aryl or acyl.

The method of the present invention is especially useful where R¹ is methyl or H, i.e., codeine or morphine, respectfully, leading to the formation of hydrocodone or hydromorphone, respectfully.

In one embodiment of the present invention, the transition metal catalysts of the present invention comprise a transition metal complex [M(PR³R⁴R⁵)ₙXₘ]ₚ, wherein M is preferably a Group 8, 9 or 10 transition metal; R³, R⁴ and R⁵ are independently selected from alkyl aryl, and alkoxyl, phenoxyl groups; X is an anion selected from the group consisting of CHO₂, CH₃CO₂, CF₃CO₂, ArCO₂, CH₃SO₃, p-tolylSO₃, HSO₄ and H₂PO₄; n is 1, 2, 3 or 4; and m is 1 or 2. These complexes are capable of polymerizing, therefore p is at least 1. When all three of the R groups are alkoxyl, phenoxyl or combinations thereof, the ligand of the complex is termed a tertiary phosphite. If at least one of the R groups is not an alkoxyl or phenoxyl, then the ligand of the complex is termed a tertiary phosphine. Preferred metals include Rh, Ru, Pd and Pt. Anions include but are not limited to CHO₂, CF₃CO₂, CH₃CO₂, ArCO₂, CH₃SO₃, p-tolylSO₃, HSO4 and H₂PO₄, R² is an alkyl or aryl group, with phenyl being preferred. Many of these catalysts are commercially available, or are easily prepared as is known in the art.

In another embodiment of the present invention, the transition metal catalysts of the present invention comprise a transition metal complex of a tertiary phosphine halide, [M(PR²₃)ₙXₘ]ₚ, wherein M is preferably a Group 8, 9 or 10 transition metal; R² is an alkyl or aryl; X is an anion selected from the group consisting of CHO₂, CH₃CO₂, CF₃CO₂, ArCO₂, CH₃SO₃, p-tolylSO₃, HSO₄ and H₂PO₄; is 1, 2, 3 or 4; and m is 1 or 2. These complexes are capable of polymerizing, therefore p is at least 1. Preferred metals include Rh, Ru, Pd and Pt. Anions include but are not limited to CHO₂, CF₃CO₂, CH₃CO₂, ArCO₂, CH₃SO₃, p-tolylSO₃, HSO₄ and H₂PO₄. R² is an alkyl or aryl group, with phenyl being preferred. Many of these catalysts are commercially available, or are easily prepared as is known in the art. It is noted that the catalysts of the formula [M(PR²₃)ₙXₘ]ₚ are a subgroup of the broader group of catalysts [M(PR³R⁴R⁵)ₙXₘ]p.

The complexes of the present invention may be comprised of solid supported tertiary phosphines/phosphites, including but not limited to polymer supported tertiary phosphines/phosphites, silical supported tertiary phosphines/phosphites and resin-bound tertiary phosphines/phosphites. In the case of solid supported tertiary phosphines/phosphites, one of the R groups typically contains a linking group connecting a phosphine/phosphite and a solid phase, as is well known in the art A non-limiting example of a solid supported tertiary phosphine useful in the present invention is the copolymer prepared from the monomer p-styryldiphenylphosphine also known as diphenyl(po-vinylphenyl)phosphinc with styrene or silical supported tertiary phosphines made from treating silica with (EtO)₃SiCH₂CH₂PPh₂, There are solid supported tertiary phosphines/phosphites that are commercially available.

In Formula I and Formula II, alkyl groups useful therein typically include those having from about 1 to about 10 carbon atoms. Aryl groups in Formula I and Formula II include phenyl and substituted phenyl groups.

In one embodiment of the invention the metal is Rh and the complex is of the formula [Rh(PR³R⁴R⁵)ₙXₘ]ₚ; wherein R³, R⁴ and R⁵ are selected from the group consisting of alkyl, aryl, alkoxyl, phenoxyl and combinations thereof; X is an anion selected from the group consisting of CHO₂, CH₃CO₂, CF₃CO₂, ArCO₂, CH₃SO₃, p-tolylSO₃, HSO₄ and H₂PO₄; n is 1, 2, 3 or 4; m is 1 or 2; and p is at least 1.

In another embodiment of the present invention the transition metal is Rh, and the metal complex is of the formula [Rh(PR¹₃)ₙY]ₚ, wherein R² is an alkyl or aryl, n is 1, 2 or 3, p is at least 1 and Y is an anion selected from the group consisting of CHO₂, CF₃CO₂, CH₃CO₂, ArCO₂, CH₃SO₃, p-tolylSO₃, HSO₄ or H₂PO₄.

In yet another embodiment of the present invention the transition metal is Ru and the metal complex is of the formula [Ru(PR³R⁴R⁵)ₙXₘ]ₚ; wherein R³, R⁴ and R⁵ are selected from the group consisting of alkyl, aryl, alkoxyl, phenoxyl and combinations thereof. X is an anion selected from the group consisting of CHO₂, CH₃CO₂, CF₃CO₂, ArCO₂, CH₃SO₃, p-tolylSO₃, HSO₄ and H₂PO₄; n is 1, 2, 3 or 4; m is 1 or 2; and p is at least 1.

In another embodiment the transition metal is Ru and the metal complex is of the formula [RuYX(PR²₃)ₙ]ₚ wherein CHO₂, CH₃CO₂, ArCO₂, CH₃SO₃, p-tolylSO₃, HSO₄ or H₂PO₄.

In another illustrative embodiment of the present invention the Rh-, Ru-, and/or Ir-complex can be bound to a tertiary copolymer of styrene, divinylbenzene, and diphenyl(p-vimylphenyl)phosphine also known as p-styryldiphenylphosphine, illustrated in Formula III:

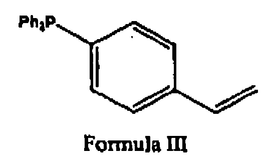

Optionally, other monomers may be substituted or added to optimize certain physical properties of the overall polymeric catalyst. IIIustrative examples include but are not limited to, ethylene dimethacrylate, p-bromostyrene, and crosslinking agents such as divinylbenzene, butadiene, diallyl maleate, diallyl phthalate, glycol dimethacrylate, and other di- or triolefins. Other phosphine containing monomers bound to the styrene ring can have, in addition to diaryl substitutions, dialkyl, branched and cyclic dialkyl, dialkoxyl or mixed substitutions or these. Illustrative examples utilizing styrene are shown in Formulas IV and V.

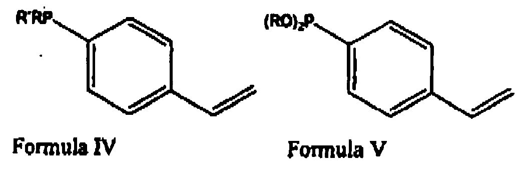

The polymeric complex makes up a composition of matter consisting essentially of an intrinsically porous solid organic styrene-divinyl benzene copolymer; a substituent phosphine group chemically bonded through phosphorus to a carbon atom of said polymer; and a Group 8, 9 or 10 metal chemically bonded to said substituent phosphine which composition is substantially insoluble in the solvent(s).

In an illustrative embodiment the polymeric support is composed of a styrene divinylbenzene copolymer containing 2 to 20 mole percent divinylbenzene, in which 0.5 to 7 mole percent, preferably 5 to 6 mole percent of the pendant phenyl groups from the copolymerized styrene contain the diphenylphosphine moiety at the para position The composition of the polymer support is 75 to 97.6 mole percent styrene, 2 to 20 percent divinylbenzene and 0.4 to 6 percent p-diphenylphosphenostyrene.

As to the amount of ruthenium complex in the catalyst, typically the Ru/pcndant atom ratio is preferably at least 0.001 and is more preferably about 0.5 to an upper limit set by the point at which the polymer support will no longer take up complex, for example about 1.2.

The polymeric complex may be made by contacting a solution of the Group 8, 9 or 10 metal salt complex in solution with the polymer support. For practical application, the metal complex is dissolved in a suitable solvent including but not limited to water, methanol, ethanol isopropanol, isobutyl alcohol, N-methylpyrrolidone, N,N-dimethylformamide, N,N-dimethylacetamide and mixtures thereof. The Group 8, 9 or 10 metal complex solution should be at least 10⁻⁶ M in the ruthenium complex but is preferably about 10⁻³ M in the ruthenium complex.

In this illustrative embodiment, the polymer portion of the catalyst is intrinsically porous. Porosity of the polymer portion of the catalyst imparts increased activity to the catalyst. With catalysts having an organic polymer portion which is not intrinsically porous, porosity may be induced into the polymer portion by solvent swelling. Combinations of the above-noted solvents can be manipulated to produce various degrees of swelling of the polymer portion of the catalyst, as is well known in the art.

The catalytic conversion of the present invention has the further economic advantage of being able to convert a salt of codeine or morphine, for example codeine hydrochloride or morphine hydrochloride, to hydrocodone or hydromorphone, respectively.

Further, the catalysts of the present invention facilitate high value metal recovery of the metal containing solid catalyst. When recovered by conventional methods known in the art, the metal of the present catalyst results in an improved recovery percentage. This further reduces material and labor costs, as well as simplifying processing/workup. For purposes herein, the term recovery is meant to encompass recovery, recycling and/or regeneration of the metal.

In the embodiments wherein the catalyst is on a fixed support, the catalyst/support can be placed in a column or container as part of a loop reactor. In a non-limiting illustrative example codeine in alcohol that is heated in a reactor can be pumped or gravity fed through a catalyst bed and cycled back to the reactor until the desired conversion to hydrocodone is produced. The advantages of this method include that many cycles (perhaps several batches) of product may be obtained with a given bed. The catalyst value is then easily recovered and sent back for reprocessing. The purification workup is simplified because the calalyst is not present in the solution. For practical reasons, the concentration of Group 8, 9 or 10 metals must be below about 10 ppm in the product. On cooling, the product would crystallize out of solution in high purity, to be recovered by filtration or centrifugation.

Many process designs, as are well known in the art, could be utilized with the process of the present invention.

The reaction of the present invention may be accomplished by any conventional process. A suitable process includes dissolving the reactant of Formula I in a suitable solvent in a reaction vessel. Suitable solvents include but are not limited to alcohols, preferably primary and secondary lower alkyl alcohols. The reaction vessel is then flushed with an inert atmosphere, typically nitrogen. The catalyst is added and the reaction mixture is refluxed under the inert atmosphere until the conversion is essentially complete, typically at least about an hour. The reaction mixture is cooled and crystals of the product are collected. The product may be purified by recrystallization in a suitable solvent as is well known in the art, or by any other suitable conventional method of purification.

In an alternative embodiment a tertiary amine, for example triethylamine, is added to the reaction mixture when the preferred Ru complex catalyst is used. The tertiary amine reduces the formation of side products, primarily the alkaloid neopine, a potential side product in reactions of the present invention utilizing the preferred Ru catalyst.

In another alternative embodiment the alkaloid compound is added to the reactants that form the catalyst and the catalyst formation reaction takes place in the presence of the alkaloid. The ability to form the catalyst and subsequently accomplish the catalytic conversion in the same reaction vessel further enhances the economy of the reaction.

### Examples.

### Comparative Example 1

Codeine, 50.00 g, was dissolved in 200 ml methanol in a three-neck flask at room temperature. The flask was equipped with a condenser and nitrogen. The flask was flushed with nitrogen for five minutes with one neck opened. The catalyst, 0.50g of RhCl(C₆H₅)₃)₃ was added to the solution. The flask was then flushed with nitrogen for another five minutes, and the open neck was closed. The reaction mixture was stirred under nitrogen and heated to reflux for four hours, then cooled to 0°C for 30 minutes. The resulting crystals were removed by filtration. The collected crystals were washed four times with 10ml cold methanol (5°C), and dried in air for one hour yielding pate yellow crystals (41.50 g. yield 83%). The filtrate was pumped down to dryness to give 6.14g yellow solid. The solid residue was dissolved in 40 ml refluxing methanol and cooled to 0°C for 30 minutes and filtered. The collected crystals were washed four times with 3 ml portions of cold methanol (5°C) and air dried for 2 hours yielding 3.41 g (6.8%) white crystals. HPLC analysis and NMR spectra confirm that the product is pure hydrocodone.

### Comparative Example 2

Morphine, 50.00 g, was suspended in 500 ml methanol in a three neck flask equipped with condenser and nitrogen input and outlet. After refluxing under nitrogen for five minutes, one neck of the flask was opened. A catalyst, 0.50g RhCl(C₆H₅)₃)₃ was added to the container. The opened neck was closed with a stopper. The reaction mixture was stirred under nitrogen and heated to reflux for 6 hours, cooled down to 0°C for 30 minutes, and filtered. The collected solid was washed four times with cold methanol (5°C) and dried in air for 20 minutes. The solid was kept under vacuum (15 mm Hg) at room temperature for 1 hour, yielding a white powder (38.83 g, yield 77.7%). The filtrate was distilled under nitrogen until only 200 ml of solution remained. It was cooled down to 0°C for 30 minutes and filtered, yielding a white powder. The product was washed twice with 20 ml each and then once with 10ml of cold methanol (5°C) dried in air for 40 minutes to yield 3.48 g of a white powder product. HPLC analysis and NMR spectra confirm product is pure hydromorphone.

### Comparative Example 3

Ruthenium dimer was prepared by refluxing 1 g RuCl₃xH₂O with 3 equivalents P(C₆H₅)₃ in EtOH (100 ml) overnight. The resulting catalyst, (Ru(P(C₆H₅)₃)₂Cl₂]₂ was obtained as a black precipitate after filtration, 63% yield.

### Comparative Example 4

The catalyst of Example 3 was reacted with codeine in MeOH in the presence of triethylamine in the ratios shown in the table below. The reaction mixtures were flushed with N₂ for 5 minutes, heated to reflux under N₂, cooled to 0°C and filtered. The recovered crystals were washed twice with 5 ml MeOH and air dried to yield white crystals.

**Table 1**

| **Starting Materials** | **[Ru(P(C₆H₅)₃)₂Cl₂]₂** | **MeON** | **NEt₃** | **Reaction time** | **Products** | **Hydrocodone/codeine/neopine (area % by HPLC)** |
|---|---|---|---|---|---|---|
| Codeine 1.0 g | 10 mg | 5 ml | 0.25 ml | 2 h | 0.85 g | 96.7/1.8/0.33 |
| Codeine 1.0 g | 10 mg | 5 ml | 0.25 ml | 3 h | 0.99 g | 94.7/0/0 |
| Codeine 2.5 g | 25 mg | 12.5 ml | 0.725 ml | 3h | 1.58 g | 100/0/0 |
| Codeine 1 g/Codeine, sulfate 0.05g | 10mg | 5 ml | 0.25ml | 3h | 0.61g | 97.4/1.0/0 |
| Codeine 1g/CodeineHCL 0.05g | 55 mg | 10ml | 0 | 3 h | 1.17g | 77.5/2.5/0.8 |
| CodeincHCl 1.1g | 100mg | 10ml | 0 | 15h | 1.16g | 81.5/11.6/0 |
| Morphine (recry) 1.0 g | 80 mg | 10ml | 0.25 | 3 days | 1.08g | Hydromorphone/morphine = 88.6/6.1 |

### Comparative Example 5

Codeine, 5.0 g, and 0.117g catalyst, Ru(P(C₆H₅)₃)₃Cl₂, were dissolved in 25 ml EtOH. The mixture was flushed with nitrogen for 5 minutes. After refluxing under nitrogen for 2 hours the reaction mixture was cooled down to 0°C and filtered. The collected crystals were washed twice with 5 ml each MeOH and dried in air for 1 hour to yield white crystals, 70% yield hydrocodone.

### Comparative Example 6

Morphine, 1.0 g, and 80 mg [Ru(P(C₆H₅)₃)₂Cl₂]₂ was suspended in 10 ml MeOH. The reaction mixture was flushed with N₂ for 3 minutes, after which 0.25 ml triethylamine was added, and the mixture flushed with N₂ for another 3 minutes. The reaction mixture was heated to reflux with stirring under N₂ for 72 hours. The resulting solid was found to be hydromorphone.

### Comparative Example 7

Codeine, 5.09 g, 25.4 mg RuCl₃xH₂O and 95.9 mg P(C₆H₅)₃ were added to 25 ml ethanol in a three neck flask. The flask was flushed with N₂ for 5 minutes. The mixture was heated until reactants dissolved, and then refluxed, under nitrogen, overnight. The resulting solution was washed twice with 5 ml each MeOH and air dried to yield 3.31 g product. Analysis determined the presence of hydrocodone and Ru catalyst.

### Comparative Example 8

Codeine-HCl, 1.1 g and 0.1 g RuCl₂(P(C₆H₅)₃)₃ were stirred in 10 ml MeOH and flushed with N₂ for 5 minutes. The reaction mixture was refluxed overnight under N₂, cooled to room temperature and dried under vacuum, yielding 1.16 g brown solid containing hydrocodone.

### Example 9

Ru Polymer Catalyst Conversion to Hydrocodone: Codeine, 50.00 g, is added to 200 ml methanol contained in a three necked flask equipped with condenser and nitrogen input and outlet The mixture is taken to reflux under nitrogen. A polymer phosphine anchored Ru catalyst, 5 g (1 mole % Ru) is added to the container. The reaction mixture is stirred under nitrogen at reflux for several hours. An additional 800 ml of methanol is added and heating is continued for 30 minutes. The polymer catalyst is recovered for recycle by filtration of the hot liquor. About 800 ml of methanol is removed from the filtrate by distillation. The liquor is then cooled to 0°C and maintained at that temperature for at least about 30 minutes. The product is collected by filtration, washed four times with cold methanol (5°C) and air dried. The product is placed under vacuum (<15 mm Hg) at room temperature for at least an hour. The product is obtained as a white powder weighing over 38 grams for a >75% yield. The purity and identity of the hydrocodone is confirmed by HPLC, H' and C¹³ NMR and MS analyses.

From the foregoing description those skilled in the art will appreciate that economical and efficient methods for the catalytic conversion of codeine or morphine into hydrocodone or hydromorphone, respectively, are provided.

## Claims

1. A method comprising catalytically converting a compound of Formula I into a compound of Formula II utilizing at least one transition metal complex of the formula [M(PR³R⁴R⁵)ₙXₘ]ₚ; wherein R¹ is H, alkyl, aryl or acyl; M is a Group 8, 9 or 10 transition metal; R³, R⁴ and R⁵ are selected from the group consisting of alkyl, aryl, alkoxyl, phenoxyl and combinations thereof; X is an anion selected from the group consisting of CHO₂, CH₃CO₂, CF₃CO₂, ArCO₂, CH₃SO₃, p-tolylSO₃, HSO₄ and H₂PO₄; n is 1, 2, 3 or 4; m is 1 or 2; and p is at least 1.

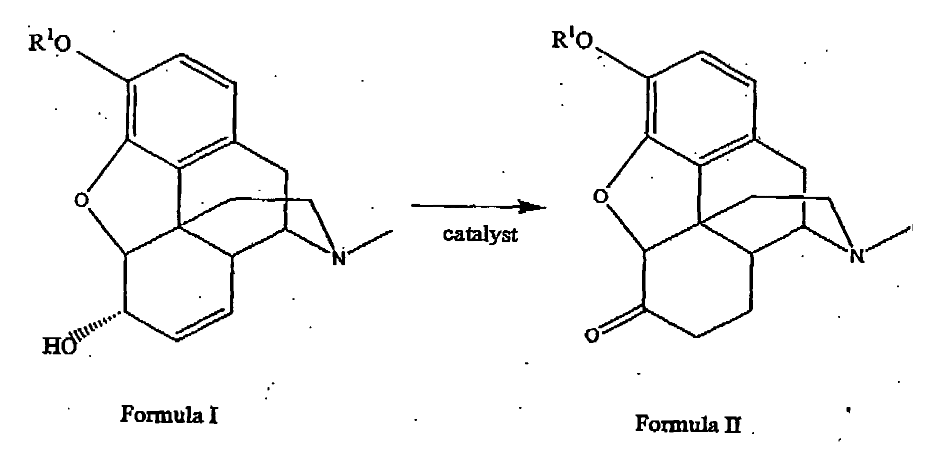

2. The method of Claim 1 wherein (PR³R⁴R⁵)ₙ is selected from the group consisting of polymer supported (PR³R⁴R⁵)ₙ, silical supported (PR³R⁴R⁵)ₙ, resin-bound (PR³R⁴R⁵)ₙ and mixtures thereof.

3. The method of Claim 1 wherein the metal complex is bound to a tertiary copolymer selected from the group consisting of styrene, divinylbenzene, and diphenyl (p-vinylphenyl)phosphine.

4. The method of Claim 2 wherein the copolymer is substituted with a monomer selected from the group consisting of ethylene dimethacrylate, p-broxctostyrene, divinylbenzene, butadiene, diallyl maleate, diallyl phthalate, glycol dimethacrylate, diolefins and triolefins.

5. The method of Claim 1 wherein the metal complex is of the formula [Rb(PR³R⁴R⁵)ₙXₘ]ₚ; wherein M is a Group 8, 9 or 10 transition metal; R³, R⁴ and R⁵ are selected from the group consisting of alkyl, aryl, alkoxyl, phenoxyl, and combinations thereof; n is 1,2, 3 or 4; m is 1 or 2; and p is at least 1.

6. The method of Claim 5 wherein n is 1, 2 or 3.

7. The method of Claim 1 wherein in the metal complex is of the formula [RuXY(PR³R⁴R⁵)ₙ]ₚ; wherein M is a Group 8, 9 or 10 transition metal; R³, R⁴ and R⁵ are selected from the group consisting of alkyl, aryl, alkoxyl, phenoxyl and combinations thereof; Y is an anion selected from the group consisting of CHO₂, CH₃CO₂, CF₃CO₂, ArCO₂, CH₃SO₃, p-tolylSO₃, HSO₄ and H₂PO; n is 1,2, 3 or 4; m is 1 or 2; and p is at least 1.

8. The method of Claim 7 wherein n=2,3 or 4 and X=Y is selected from the group consisting of CHO₂, CF₃CO₂, CH₃CO₂, ArCO₂, CH₃SO₃, p-tolylSO₃, HSO₄ and H₂PO₄.

9. The method according to any one of the preceding claims wherein R³ = R⁴ = R⁵ = R² and the metal complex is of the formula [M(PR²₃)ₙXₘ]ₚ, wherein R² is an alkyl or aryl.

10. The method of Claim 9 wherein the metal complex is of the formula [Rh(PR²₃)ₙX]ₚ, wherein R² is an alkyl or aryl; n is 1, 2 or 3; and p is at least 1.

11. The method of Claim 10 wherein the metal complex having the formula [Rh(PR²₃)ₙY]ₚ, wherein n is 1, 2 or 3; p is at least 1; and Y is selected from the group consisting of CHO₂, CF₃CO₂, CH₃CO₂, ArCO₂, CH₃SO₃, p-tolylSO₃, HSO₄ and H₂PO₄.

12. The method of Claim 9 wherein the metal complex is of the formula [RuYX(PR²₃)ₙ]ₚ wherein R² is an alkyl or aryl and wherein n=2,3 or 4 and X=Y is selected from the group consisting of CHO₂, CF₃CO₂, CH₃CO₂, ArCO₂, CH₃SO₃, p-tolylSO₃, HSO₄ and H₂PO₄.

13. The method according to claim 1 for producing hydrocodone wherein Formula I is codeine and Formula II is hydrocodone.

14. The method of Claim 13 wherein R³ = R⁴ = R⁵ = R² and the metal complex is of the formula [Rh(PR²₃)ₙY]ₚ, wherein R² is an alkyl or aryl; n is 1, 2 or 3 and p is at least 1; and Y is selected from the group consisting of CHO₂, CF₃CO₂, CH₃CO₂, ArCO₂, CH₃SO₃, p-tolylSO₃, HSO₄ and H₂PO₄.

15. The method of Claim 13 wherein the metal complex is of the formula [RuYX(PR²₃)ₙ]ₚ wherein R² is an alkyl or aryl and wherein n=2,3 or 4, p is at least 1 and X=X is selected from the group consisting of CHO₂, CF₃CO₂, CH₃CO₂, ArCO₂, CH₃SO₃, P-tolylSO₃, HSO₄ and H₂PO₄.

16. The method according to claim 9 for producing hydromorphone wherein Formula I is morphine and Formula II is hydromorphone.

17. The method of Claim 13 or claim 16 wherein the metal complex is of the formula [Rh(PR²₃)ₙX]ₚ, wherein R² is an alkyl or aryl; n is 1, 2 or 3; and p is at least 1.

18. The method of Claim 16 wherein the metal complex is of the formula [Rh(PR²₃)ₙY]ₚ, wherein R² is an alkyl or aryl; n is 1, 2 or 3; p is at least 1; and Y is selected from the group consisting of CF₃CO₂, CH₃CO₂, ArCO₂, CH₃SO₃, p-tolylSO₃, HSO₄ and H₂PO₄.

19. The method of Claim 16 wherein the metal complex is of the formula [RuYX(PR²₃)ₙ]ₚ wherein R² is an alkyl or aryl and wherein n=2,3 or 4, p is at least 1 and X=Y is selected from the group consisting of CF₃CO₂, CH₃CO₂, ArCO₂, CH₃SO₃, p-tolylSO₃, HSO₄ and H₂PO₄.

20. The method of claim 1 additionally comprising the step of recovering the metal containing solid catalyst.

21. The method of Claim 1, 9 or 20 wherein R¹ is H or CH₃.

22. The method of Claim 1, 13 or 20 wherein (PR³R⁴R⁵)ₙ is supported by a solid.

23. The method of Claim 13 or 20 wherein (pR³R⁴R⁵)ₙ is selected from the group consisting of polymer supported (PR³R⁴R⁵)ₙ, silical supported (PR³R⁴R⁵)ₙ, resin-bound (PR³R⁴R⁵)ₙ and mixtures thereof.

## Patentansprüche

1. Verfahren, umfassend das katalytische Überführen einer Verbindung nach Formel 1 zu einer Verbindung nach Formel 11 unter Verwendung mindestens eines Übergangsmetallkomplexes der Formel [M(PR ³R⁴R⁵)ₙXₘ]ₚ; wobei R¹ gleich H, Alkyl, Aryl oder Acyl ist; M ein Übergangsmetall der Gruppe 8, 9 oder 10 ist; R³, R⁴ und R⁵ ausgewählt sind aus der Gruppe, bestehend aus Alkyl, Aryl, Alkoxyl, Phenoxyl, und Kombinationen davon; X ein Anion ist, ausgewählt aus der Gruppe, bestehend aus CHO₂, CH₃CO₂, CF₃CO₂, ArCO₂, CH₃SO₃, p-Tolyl-SO₃, HSO₄ und H₂PO₄; n gleich 1, 2, 3 oder 4 ist; m gleich 1 oder 2 ist; und p mindestens 1 ist.

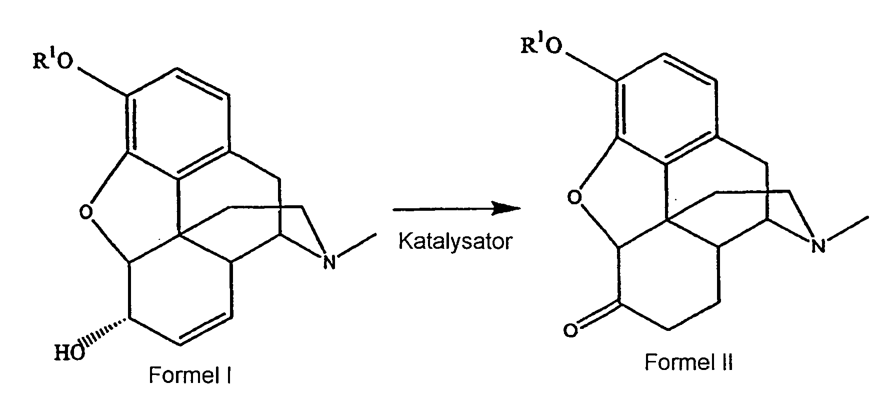

2. Verfahren nach Anspruch 1, wobei (PR³R⁴R⁵)ₙ ausgewählt ist aus der Gruppe, bestehend aus Polymer-gestütztem (PR³R⁴R⁵)ₙ, Silica-gestütztem (PR³R⁴R⁵)ₙ, Harz-gebundenem (PR³R⁴R⁵)ₙ, und Gemischen davon.

3. Verfahren nach Anspruch 1, wobei der Metallkomplex an ein tertiäres Copolymer gebunden ist, ausgewählt aus der Gruppe, bestehend aus Styrol, Divinylbenzol, und Diphenyl(p-vinylphenyl)phosphin.

4. Verfahren nach Anspruch 2, wobei das Copolymer mit einem Monomer, ausgewählt aus der Gruppe, bestehend aus Ethylendimethacrylat, p-Bromstyrol, Divinylbenzol, Butadien, Diallylmaleat, Diallylphthalat, Glycoldimethacrylat, Diolefinen und Triolefinen, substituiert ist.

5. Verfahren nach Anspruch 1, wobei der Metallkomplex die Formel [Rh(PR³R⁴R⁵)ₙXₘ]ₚ aufweist; wobei M ein Übergangsmetall der Gruppe 8, 9 oder 10 ist; R³, R⁴ und R⁵ ausgewählt sind aus der Gruppe, bestehend aus Alkyl, Aryl, Alkoxyl, Phenoxyl, und Kombinationen davon; n gleich 1, 2, 3 oder 4 ist; m gleich 1 oder 2 ist; und p mindestens 1 ist.

6. Verfahren nach Anspruch 5, wobei n gleich 1, 2 oder 3 ist.

7. Verfahren nach Anspruch 1, wobei der Metallkomplex die Formel [RuXY(PR³R⁴R⁵)ₙ]ₚ aufweist; wobei M ein Übergangsmetall der Gruppe 8, 9 oder 10 ist; R³, R⁴ und R⁵ ausgewählt sind aus der Gruppe, bestehend aus Alkyl, Aryl, Alkoxyl, Phenoxyl, und Kombinationen davon; Y ein Anion ist, ausgewählt aus der Gruppe, bestehend aus CHO₂, CH₃CO₂, CF₃CO₂, ArCO₂, CH₃SO₃, p-Tolyl-SO₃, HSO₄ und H₂PO; n gleich 1, 2, 3 oder 4 ist; m gleich 1 oder 2 ist; und p mindestens 1 ist.

8. Verfahren nach Anspruch 7, wobei n=2, 3 oder 4 und X=Y ausgewählt ist aus der Gruppe, bestehend aus CHO₂, CF₃CO₂, CH₃CO₂, ArCO₂, CH₃SO₃, p-Tolyl-SO₃, HSO₄ und H₂PO₄.

9. Verfahren nach einem der vorhergehenden Ansprüche, wobei R³ = R⁴ = R⁵ = R², und der Metallkomplex die Formel [M(PR²₃)ₙXₘ]ₚ aufweist, worin R² ein Alkyl oder Aryl ist.

10. Verfahren nach Anspruch 9, wobei der Metallkomplex die Formel [Rh(PR²₃)ₙX]ₚ aufweist, worin R² ein Alkyl oder Aryl ist; n gleich 1, 2 oder 3 ist; und p mindestens 1 ist.

11. Verfahren nach Anspruch 10, wobei der Metallkomplex die Formel [Rh(PR²₃)ₙY]ₚ aufweist, worin n gleich 1, 2 oder 3 ist; p mindestens 1 ist; und Y ausgewählt ist aus der Gruppe, bestehend aus CHO₂, CF₃CO₂, CH₃CO₂, ArCO₂, CH₃SO₃, p-Tolyl-SO₃, HSO₄ und H₂PO₄.

12. Verfahren nach Anspruch 9, wobei der Metallkomplex die Formel [RuYX(PR²₃)ₙ]ₚ aufweist, worin R² ein Alkyl oder Aryl ist; und wobei n gleich 2, 3 oder 4 ist; und X=Y ausgewählt ist aus der Gruppe, bestehend aus CHO₂, CF₃CO₂, CH₃CO₂, ArCO₂, CH₃SO₃, p-Tolyl-SO₃, HSO₄ und H₂PO₄.

13. Verfahren nach Anspruch 1 zur Herstellung von Hydrocodon, wobei Formel I Codein ist und Formel II Hydrocodon ist.

14. Verfahren nach Anspruch 13, wobei R³ = R⁴ = R⁵ = R², und der Metallkomplex die Formel [Rh(PR²₃)ₙY]ₚ aufweist, worin R² ein Alkyl oder Aryl ist; n gleich 1, 2 oder 3 ist; und p mindestens 1 ist; und Y ausgewählt ist aus der Gruppe, bestehend aus CHO₂, CF₃CO₂, CH₃CO₂, ArCO₂, CH₃SO₃, p-Tolyl-SO₃, HSO₄ und H₂PO₄.

15. Verfahren nach Anspruch 13, wobei der Metallkomplex die Formel [RuYX(PR²₃)ₙ]ₚ aufweist, worin R² ein Alkyl oder Aryl ist; und worin n gleich 2, 3 oder 4 ist; p mindestens 1 ist; und X=Y ausgewählt ist aus der Gruppe, bestehend aus CHO₂, CF₃CO₂, CH₃CO₂, ArCO₂, CH₃SO₃, p-Tolyl-SO₃, HSO₄ und H₂PO₄.

16. Verfahren nach Anspruch 9 zur Herstellung von Hydromorphon, wobei Formel 1 Morphin ist und Formel 11 Hydromorphon ist.

17. Verfahren nach Anspruch 13 oder 16, wobei der Metallkomplex die Formel [Rh(PR²₃)ₙX]ₚ aufweist, worin R² ein Alkyl oder Aryl ist; n gleich 1, 2 oder 3 ist; und p mindestens 1 ist.

18. Verfahren nach Anspruch 16, wobei der Metallkomplex die Formel [RH(PR²₃)ₙY]ₚ aufweist, worin R² ein Alkyl oder Aryl ist; n gleich 1, 2 oder 3 ist; p mindestens 1 ist; und Y ausgewählt ist aus der Gruppe, bestehend aus CF₃CO₂, CH₃CO₂, ArCO₂, CH₃SO₃, p-Tolyl-SO₃, HSO₄ und H₂PO₄.

19. Verfahren nach Anspruch 16, wobei der Metallkomplex die Formel [RuYX(PR²₃)ₙ]ₚ aufweist, worin R² ein Alkyl oder Aryl ist; und worin n gleich 2, 3 oder 4 ist; p mindestens 1 ist; und X=Y ausgewählt ist aus der Gruppe, bestehend aus CF₃CO₂, CH₃CO₂, ArCO₂, CH₃SO₃, p-Tolyl-SO₃, HSO₄ und H₂PO₄.

20. Verfahren nach Anspruch 1, zusätzlich umfassend den Schritt, den Metallenthaltenden festen Katalysator zurückzugewinnen.

21. Verfahren nach Anspruch 1, 9 oder 20, wobei R¹ gleich H oder CH₃ ist.

22. Verfahren nach Anspruch 1, 13 oder 20, wobei (PR³R⁴R⁵)ₙ durch einen Feststoff gestützt ist.

23. Verfahren nach Anspruch 13 oder 20, wobei (PR³R⁴R⁵)ₙ ausgewählt ist aus der Gruppe, bestehend aus Polymer-gestütztem (PR³R⁴R⁵)ₙ, Silica-gestütztem (PR³R⁴R⁵)ₙ, Harz-gebundenem (PR³R⁴R⁵)ₙ, und Gemischen davon.

## Revendications

1. Procédé comprenant la conversion catalytique d'un composé de Formule I en un composé de Formule II utilisant au moins un complexe de métal de transition de formule [M(PR³R⁴R⁵)ₙXₘ]ₚ ; où R¹ est H, un groupe alkyle, aryle ou acyle ; M est un métal de transition du Groupe 8, 9 ou 10 ; R³, R⁴ et R⁵ sont choisis dans le groupe formé par les groupes alkyle, aryle, alkoxyle, phénoxyle et leurs associations ; X est un anion choisi dans le groupe formé par CHO₂, CH₃CO₂, CF₃CO₂, ArCO₂, CH₃SO₃, *p*-tolylSO₃, HSO₄ et H₂PO₄; n est 1, 2, 3 ou 4 ; m est 1 ou 2 ; et p est au moins 1.

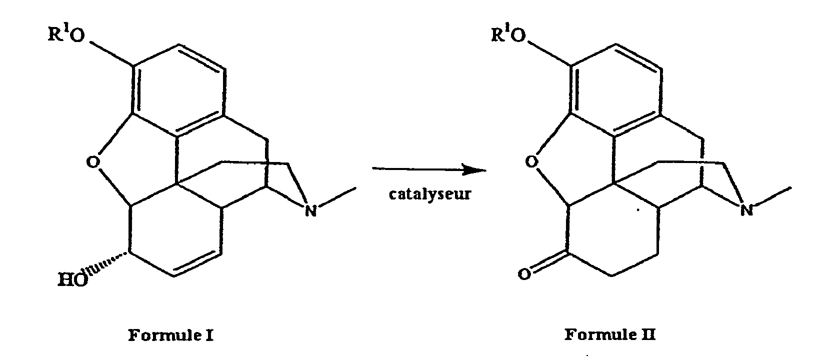

2. Procédé selon la revendication 1, dans lequel (PR³R⁴R⁵)ₙ est choisi dans le groupe formé par (PR³R⁴R⁵)ₙ supporté sur polymère, (PR³R⁴R⁵) ₙ supporté sur silice, (PR³R⁴R⁵)ₙ lié à une résine, et leurs mélanges.

3. Procédé selon la revendication 1, dans lequel le complexe métallique est lié à un copolymère tertiaire choisi dans le groupe consistant en styrène, divinylbenzène et diphényl(*p*-vinylphényl)phosphine.

4. Procédé selon la revendication 2, dans lequel le copolymère est substitué par un monomère choisi dans le groupe formé par le diméthacrylate d'éthylène, le *p*-bromo-styrène, le divinylbenzène, le butadiène, le maléate de diallyle, le phtalate de diallyle, le diméthacrylate de glycol, des dioléfines et des trioléfines.

5. Procédé selon la revendication 1, dans lequel le complexe métallique répond à la formule [RH(PR³R⁴R⁵)ₙXₘ]ₚ ; où M est un métal de transition du Groupe 8, 9 ou 10 ; R³, R⁴ et R⁵ sont choisis dans le groupe formé par les groupes alkyle, aryle, alkoxyle, phénoxyle et leurs associations ; n est 1, 2, 3 ou 4 ; m est 1 ou 2 ; et p est au moins 1.

6. Procédé selon la revendication 5, dans lequel n est 1, 2 ou 3.

7. Procédé selon la revendication 1, dans lequel le complexe métallique répond à la formule [RuXY(PR³R⁴R⁵)ₙ]ₚ; où M est un métal de transition du Groupe 8, 9 ou 10 ; R³, R⁴ et R⁵ sont choisis dans le groupe formé par les groupes alkyle, aryle, alkoxyle, phénoxyle et leurs associations ; Y est un anion choisi dans le groupe formé par CHO₂, CH₃CO₂, CF₃CO₂, ArCO₂, CH₃SO₃, *p*-tolylSO₃, HSO₄ et H₂PO ; n est 1, 2, 3 ou 4 ; m est 1 ou 2 ; et p est au moins 1.

8. Procédé selon la revendication 7, dans lequel n = 2, 3 ou 4 et X = Y et est choisi dans le groupe formé par CHO₂, CF₃CO₂, CH₃CO₂, ArCO₂, CH₃SO₃, *p*-tolylSO₃, HSO₄ et H₂PO₄.

9. Procédé selon l'une quelconque des revendications précédentes, dans lequel R³ = R⁴ = R⁵ = R² et le complexe métallique répond à la formule [M(PR²₃)ₙXₘ]ₚ où R² est un groupe alkyle ou aryle.

10. Procédé selon la revendication 9, dans lequel le complexe métallique répond à la formule [Rh(PR²₃)ₙX]ₚ où R² est un groupe alkyle ou aryle ; n est 1, 2 ou 3 ; et p est au moins 1.

11. Procédé selon la revendication 10, dans lequel le complexe métallique répond à la formule [Rh(PR²₃)ₙY]ₚ où n est 1, 2 ou 3 ; p est au moins 1 ; et Y est choisi dans le groupe formé par CHO₂, CF₃CO₂, CH₃CO₂, ArCO₂, CH₃SO₃, *p*-tolylSO₃, HSO₄ et H₂PO₄.

12. Procédé selon la revendication 9, dans lequel le complexe métallique répond à la formule [RuYX(PR²₃)ₙ]ₚ où R² est un groupe alkyle ou aryle et où n = 2, 3 ou 4 et X = Y et est choisi dans le groupe formé par CHO₂, CF₃CO₂, CH₃CO₂, ArCO₂, CH₃SO₃, *p*-tolylSO₃, HSO₄ et H₂PO₄.

13. Procédé selon la revendication 1 pour la production d'hydrocodone, dans lequel la Formule I est la codéine et la Formule II est l'hydrocodone.

14. Procédé selon la revendication 13, dans lequel R³ = R⁴ = R⁵ = R² et le complexe métallique répond à la formule [Rh(PR²₃)ₙY]ₚ où R² est un groupe alkyle ou aryle ; n est 1, 2 ou 3 et p est au moins 1 ; et Y est choisi dans le groupe formé par CHO₂, CF₃CO₂, CH₃CO₂, ArCO₂, CH₃SO₃, *p*-tolylSO₃, HSO₄ et H₂PO₄.

15. Procédé selon la revendication 13, dans lequel le complexe métallique répond à la formule [RuYX(PR²₃)ₙ]ₚ où R² est un groupe alkyle ou aryle et où n = 2, 3 ou 4, p est au moins 1 et X = Y et est choisi dans le groupe formé par CHO₂, CF₃CO₂, CH₃CO₂, ArCO₂, CH₃SO₃, *p*-tolylSO₃, HSO₄ et H₂PO₄.

16. Procédé selon la revendication 9 pour la production d'hydromorphone, dans lequel la Formule I est la morphine et la Formule II est l'hydromorphone.

17. Procédé selon la revendication 13 ou la revendication 16, dans lequel le complexe métallique répond à la formule [Rh(PR²₃)ₙX]ₚ où R² est un groupe alkyle ou aryle ; n est 1, 2 ou 3 ; et p est au moins 1.

18. Procédé selon la revendication 16, dans lequel le complexe métallique répond à la formule [Rh(PR²₃)ₙY]ₚ où R² est un groupe alkyle ou aryle ; n est 1, 2 ou 3 ; p est au moins 1 ; et Y est choisi dans le groupe formé par CF₃CO₂, CH₃CO₂, ArCO₂, CH₃SO₃, *p*-tolylSO₃, HSO₄ et H₂PO₄.

19. Procédé selon la revendication 16, dans lequel le complexe métallique répond à la formule [RuYX(PR²₃)ₙ]ₚ où R² est un groupe alkyle ou aryle et où n = 2, 3 ou 4, p est au moins 1 et X = Y et est choisi dans le groupe formé par CF₃CO₂, CH₃CO₂, ArCO₂, CH₃SO₃, *p*-tolylSO₃, HSO₄ et H₂PO₄.

20. Procédé selon la revendication 1, comprenant de plus l'étape de récupération du catalyseur solide contenant le métal.

21. Procédé selon la revendication 1, 9 ou 20, dans lequel R¹ est H ou CH₃.

22. Procédé selon la revendication 1, 13 ou 20, dans lequel (PR³R⁴R⁵)ₙ est supporté par un solide.

23. Procédé selon la revendication 13 ou 20, dans lequel (PR³R⁴R⁵)ₙ est choisi dans le groupe formé par (PR³R⁴R⁵)ₙ supporté sur polymère, (PR³R⁴R⁵)ₙ supporté sur silice, (PR³R⁴R⁵)ₙ lié à une résine, et leurs mélanges.
